# EUROPEAN PATENT APPLICATION

(11) **EP 4 563 139 A1**
(43) Date of publication of application: **04.06.2025**
(21) Application number: 22953257.7
(22) Date of filing: 15.09.2022
(51) Int. Cl.: A61K 8/02, A61K 8/25, A61Q 19/00

(54) **NANO-FREE SILICA PARTICLES AND PREPARATION METHOD THEREFOR**

(30) Priority: 27.07.2022 KR 20220093418
(71) Applicant: Sunjin Beauty Science Co., Ltd., Seoul 08588 (KR)
(72) Inventor: LEE, Sung Ho, Seoul 08089 (KR); KIM, Yeong Gi, Seocheon-gun Chungcheongnam-do 33666 (KR); PARK, Hyun Woong, Gunsan-si Jeollabuk-do 54139 (KR)
(74) Representative: Bockhorni & Brüntjen Partnerschaft Patentanwälte mbB
(86) International application number: PCT/KR2022/013754
(87) International publication number: WO 2024/025032

(57) **Abstract**

The present invention relates to nano-free silica particles and a method for preparing the same, and particularly, to silica particles, as spherical silica for cosmetics having a diameter of 1 to 30 µm obtained using water glass, characterized in that they are nano free as a result of measurement with a scanning electron microscope (SEM).

Since nanomaterials are not present in the cosmetics containing silica particles according to the present invention, it is possible to prevent nano harmfulness, and improve the feeling of use by increasing the flowability.

## Description

### [Technical Field]

The present invention relates to nano-free silica particles and a method for preparing the same, and more particularly, to nano-free silica particles, as spherical silica for cosmetics with a diameter of 1 to 30 µm obtained using water glass, which can prevent nano harmfulness, and can improve the feeling of use of cosmetics due to low friction coefficient and improved flowability of cosmetics, and a method for preparing the same.

### [Background Art]

The content described below merely provides background information only related to the present invention and does not constitute the conventional art.

Silica, as an inorganic oxide of siloxane and silanes, is also called silicon dioxide or silicic anhydride, and is a combination of silicon and oxygen. As the main component of rocks or earth and sand, it is contained in very small amounts in the state of ion phase colloidal phase in natural water, and is also a major component of quartz, crystal, agate, and opal.

In cosmetics, silica particles may give a help in clean pore care by controlling the secretion of sebum in the skin. In addition, when the powder component used in cosmetics absorbs moisture, the silica particles may be used as a raw material to prevent a phenomenon such as entanglement or formation of lumps due to moisture. In addition, the silica particles may exhibit an effect in which they are used in a scrub cosmetic composition to remove harmful substances in skin metabolism, secretions, contaminants attached from the outside, bacteria, makeup substances, etc., an effect in which they stimulate nerves and blood vessels that are distributed in the dermis and subcutaneous tissue of the skin and thus perform a blood flow promotion action to prevent fine wrinkles, and a peeling effect of helping to easily remove keratinized keratin and thus preventing thickening of the stratum corneum so that the epidermis is kept healthy. In addition, the silica particles may also be used as a feeling of use improving agent in makeup products.

In an existing silica preparation method, silica particles are prepared using a method of growing silica particles by slowly adding a weak acid to tetramethyl orthosilicate (TMOS) and tetraethyl orthosilicate (TEOS). However, although this method may allow silica particles to be prepared relatively easily, it has the disadvantage of being expensive compared to a method of using water glass.

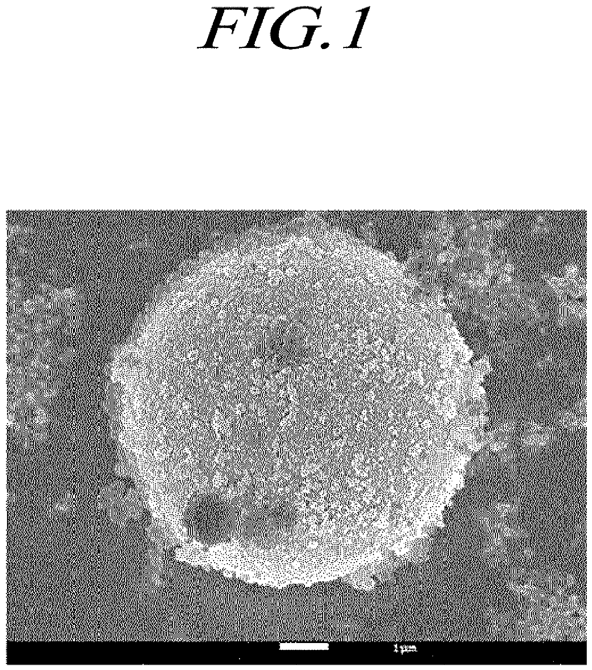

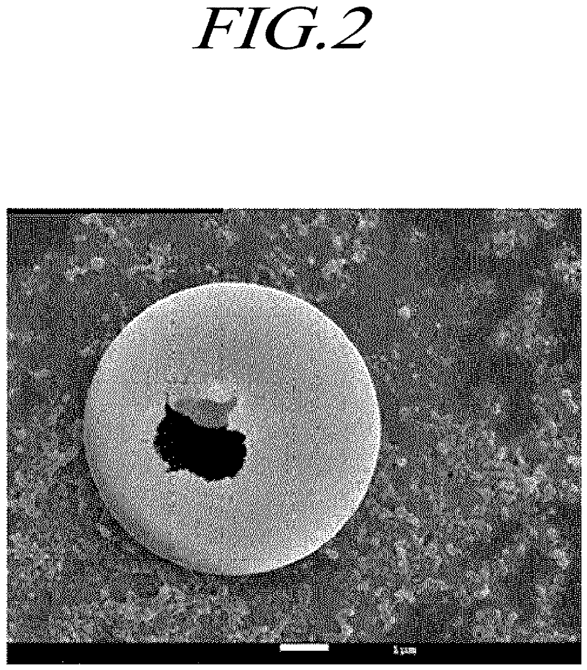

Further, since the reactivity is high when silica particles are prepared by adding acid using water glass instead of TMOS or TEOS, fine nanoparticles are instantaneously generated, and it is not only difficult to control the size of the particles, but also many nanoparticles are attached to the surface of the generated particles (FIGS. 1 and 2) so that the coefficient of friction increases due to the rough surface to result in a poor feeling of use in cosmetics. Further, considering the adverse effect of nanoparticles on the living body, a method for manufacturing new silica particles that do not contain nanoparticles is required. That is, nanomaterials may be formed in the process of manufacturing silica particles using a sodium silicate solution. When nanomaterials are contained as cosmetic ingredients, they may cause nano harmfulness and reduce the feeling of use of cosmetics.

Regarding nanomaterials, the European Community (EC) Cosmetic Regulations 1223/2009 require that insoluble or bio-persistent materials in the range of 1 to 100 nm should be stipulated as nanomaterials, and whether the nanomaterials are present or not should be reported to the Commission before cosmetics are released, and all ingredients in the form of nanomaterials should be specified in the ingredient list. In addition, the Recommendation 2011/696 on nanoparticles published in 2011 recommends that, as a result of scanning electron microscope (SEM) observation, when the number of particles with a size of 100 nm or less is 50% or less, it may be called nano free. This means that assuming that the average diameter of the particles is 10 microns (µm), particles of 100 nanometers or less are contained only in an extremely small amount of not more than one millionth by weight or volume ratio.

The present inventors were able to obtain 'nano-free silica' by controlling important process variables in a silica particle manufacturing method using water glass (Na₂SiO₃ aqueous solution).

### [Disclosure]

### [Technical Problem]

The technical problem to be achieved by the present invention is to solve the conventional problems, and an object of the present invention to provide nano-free silica spherical particles which can prevent nano harmfulness while preparing spherical silica particles for cosmetics with a diameter of 1 to 30 µm using water glass, and which can improve the feeling of use of cosmetics due to low friction coefficient and improved flowability of cosmetics, and a method for preparing the same.

### [Technical Solution]

The present invention provides, as spherical silica for cosmetics having a diameter of 1 to 30 µm obtained using water glass,
silica particles characterized in that they are nano free as a result of measurement with a scanning electron microscope (SEM).

Preferably, the silica particles have a friction coefficient (gf) of 160 gf or less, or an Avalanche Energy of 20 mj/kg or less.

Furthermore, the present invention provides a method for preparing silica particles, the method comprising: a first step of preparing a mixed solution by adding an oil and an emulsifier to water glass or a mixture of water glass and water and stirring the mixture;
a second step of adding a weak acid or a weak acid aqueous solution to the mixed solution to perform reaction;
a third step of adding a strong acid aqueous solution to a reaction product of the second step and performing reaction; and
a fourth step of drying and pulverizing a resulting product of the third step.

Preferably, the oil is liquid paraffin, light oil, or kerosene.

Preferably, the emulsifier has a Hydrophilic Lipophilic Balance (HLB) of 4 to 6.

Specifically, the weak acid may be one or more selected from the group consisting of acetic acid, carbonic acid, citric acid, and lactic acid.

Preferably, the strong acid aqueous solution is added so that the final aqueous solution has a pH of 5 to 7.

Preferably, the addition of the weak acid aqueous solution is to sequentially input two or more aqueous solutions having different concentrations from a lower concentration one.

Preferably, the addition of the strong acid aqueous solution is to sequentially input two or more aqueous solutions having different concentrations from a lower concentration one.

Furthermore, the present invention provides silica particles prepared by the method for preparing silica particles described above.

Furthermore, the present invention provides cosmetics containing the silica particles described above.

### [Advantageous Effects]

According to the present invention, it is possible to provide, as spherical silica for cosmetics with a diameter of 1 to 30 µm obtained using water glass, nano-free silica particles which can prevent nano harmfulness, and can improve the feeling of use of cosmetics due to low friction coefficient and improved flowability of cosmetics, and a method for preparing the same.

### [Description of Drawings]

FIG. 1 is a scanning electron micrograph of particles after weak acid treatment of water glass according to the conventional art.
FIG. 2 is a scanning electron micrograph of particles broken during acid treatment with strong acid according to the conventional art.
FIG. 3 is a flowchart showing a silica preparation process according to one embodiment of the present invention.

### [Best Mode]

Hereinafter, the present invention will be described in detail so that those skilled in the art to which the present invention pertains can easily carry out embodiments of the present invention with reference to the accompanying drawings. However, the present invention may be embodied in various different forms and is not limited to the embodiments described herein.

Throughout the present specification, when a certain part is said to "include" a certain component, it means that it may further include another component without excluding another component unless specifically stated otherwise.

The present invention provides, as spherical silica for cosmetics having a diameter of 1 to 30 µm obtained using water glass,
silica particles characterized in that they are nano free as a result of measurement with a scanning electron microscope (SEM).

As a result of scanning electron microscope (SEM) observation in the present invention, "nano free" is one suitable for the nano free standard set by the European Cosmetics Commission, and is when the number of particles with a size of 100 nm or less is 50% or less as a result of scanning electron microscope observation, and this means that particles of 100 nanometers or less are contained only in an extremely small amount of not more than one millionth by weight or volume ratio.

Preferably, the silica particles according to the present invention have a friction coefficient (gf) of 160 gf or less, or an Avalanche Energy of 20 mj/kg or less. In this case, the friction coefficient of the cosmetics is low and the flowability thereof is improved so that the feeling of use of the cosmetics may be improved.

The silica particles according to the present invention are nano-free silica particles obtained through water glass, and when contained in cosmetics, nano harmfulness may be fundamentally prevented and the feeling of use may be improved.

The silica particles according to the present invention may be prepared through a method for preparing silica particles, the method comprising: a first step of preparing a mixed solution by adding an oil and an emulsifier to water glass or a mixture of water glass and water and stirring the mixture;
a second step of adding a weak acid or a weak acid aqueous solution to the mixed solution to perform reaction;
a third step of adding a strong acid aqueous solution to a reaction product of the second step and performing reaction; and
a fourth step of drying and pulverizing a resulting product of the third step.

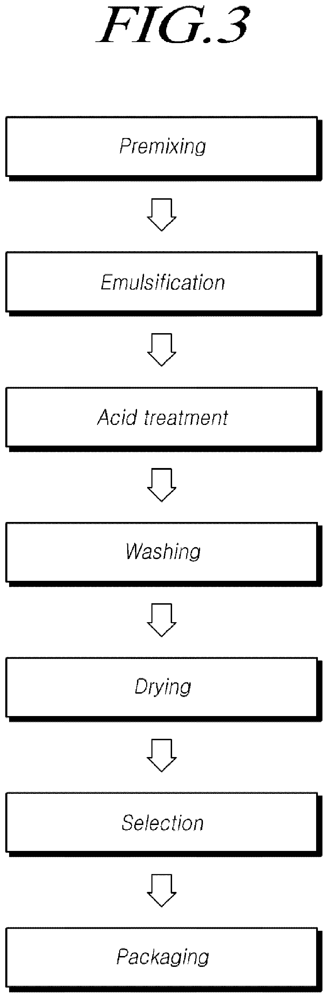

Hereinafter, the technical configuration for each preparation step of the above-described method for preparing silica particles will be reviewed in more detail with reference to FIG. 3.

In the method for preparing silica particles according to the present invention, only water glass can be used as a raw material, and can be diluted with water and used if necessary. At this time, the amount of water can be arbitrarily adjusted, and preferably, water can be used in an amount of 1 to 50% by weight in the mixed solution with water glass. As the amount of water increases, the porosity of silica to be prepared also increases.

In the present invention, an oil and an emulsifier are added to water glass or a mixture of water glass and water to prepare a lipophilic emulsion (first step). The oil that can be used at this time is preferably hydrocarbon oil, and specifically, various types of oil such as liquid paraffin, light oil, kerosene, polybutene, and the like may be used, and it is preferable to use liquid paraffin or kerosene. In this case, safety problems such as fire, and economic feasibility may be improved.

Further, the emulsifier preferably has a Hydrophilic Lipophilic Balance (HLB) of 4 to 6, and specific examples thereof preferably include one or more selected from the group consisting of sorbitan stearate, sorbitan isostearate, sorbitan oleate, sorbitan linoleate, and polyglyceryl polylinoleate. The emulsifier may be used in an amount of 0.1 to 20 parts by weight based on 100 parts by weight of oil. If the amount of the emulsifier is too small, the uniformity of the particles becomes poor since a sufficiently uniform emulsion cannot be prepared, and if the amount of the emulsifier is too large, it is impossible to obtain silica particles of a desired size since the size of the particles becomes too small.

When the oil is mixed, the ratio of the aqueous phase and the oil phase may be arbitrarily adjusted, preferably, the aqueous phase, which is the inner phase, is contained in an amount of 30 to 80 parts by weight, more preferably 50 to 70 parts by weight. Productivity and homogeneity of silica particles may be satisfied at the same time.

The method for preparing silica particles according to the present invention comprise a second step of adding a weak acid or a weak acid aqueous solution to the mixed solution of the first step to perform reaction.

The weak acid is not particularly limited, and specifically, one or more selected from the group consisting of acetic acid, carbonic acid, citric acid, and lactic acid may be used. When the weak acid is used as a weak acid aqueous solution, the concentration of the aqueous solution may be arbitrarily adjusted, and specifically, it may be prepared and used as an aqueous solution of 1 to 50% by weight, preferably 1 to 30% by weight. The weak acid or weak acid aqueous solution may be added and used in an amount of 0.5 to 10 times the oil input amount. Preferably, the weak acid aqueous solution is prepared in two or more concentrations to add a lower concentration weak acid aqueous solution, and then sequentially add a higher concentration weak acid aqueous solution. In this case, since the silica particles to be prepared has smoother surface and low friction coefficient, the flowability thereof may be further improved.

The method for preparing silica particles according to the present invention comprises a third step of adding a strong acid aqueous solution to a reaction product of the second step to perform reaction.

The strong acid may be specifically sulfuric acid, hydrochloric acid, or nitric acid, and when the strong acid is used as a strong acid aqueous solution, the aqueous solution may be prepared and used at a concentration of 1 to 20% by weight. The strong acid aqueous solution is preferably added in such an amount that the pH of the final aqueous solution is 5 to 7. In this case, it may be possible to prevent nanoparticles from being generated as the silica particles to be prepared are burst. Preferably, the strong acid aqueous solution is prepared in two or more concentrations to add a lower concentration strong acid aqueous solution, and then sequentially add a higher concentration strong acid aqueous solution. In this case, since the silica particles to be prepared has smoother surface and low friction coefficient, the flowability thereof may be further improved.

The method for preparing silica particles according to the present invention comprises a fourth step of drying and pulverizing silica, which is a resulting product of the third step. Known methods may be applied to the drying and pulverizing.

The method for preparing silica particles according to the present invention may prepare silica particles which can prevent nano harmfulness, can improve the feeling of use of cosmetics due to low friction coefficient and improved flowability, and have high productivity by preventing the generation of nanoparticles while preparing spherical silica particles for cosmetics of 1 to 30 µm using water glass.

Hereinafter, the present invention will be described in detail through Examples. However, the following Examples are only to illustrate the present invention, and the present invention is not limited by the following Examples.

### [Example 1]

After mixing 300 kg of sodium silicate and 30 kg of water, the mixture was stirred at room temperature to make it uniform. After a mixed solution of 200 kg of liquid paraffin and 20 kg of sorbitan isostearate as an emulsifier was added thereto, the mixture was stirred at room temperature to produce a water-in-oil emulsion. Particles were formed by slowly adding 700 kg of an aqueous solution containing 5% by weight of acetic acid thereto while being careful not to break the particles.

An aqueous solution containing 10% by weight of hydrochloric acid was slowly added thereto to adjust the pH of the final solution to 5 to 7 so that the particles were solidified hard. Only precipitated silica particles were taken, washed, dried, and sieved to prepare nano-free silica. As a result of photographing the prepared silica particles into a scanning electron micrograph, a nano-free state was shown.

### [Example 2]

After mixing 300 kg of sodium silicate and 30 kg of water, the mixture was stirred at room temperature to make it uniform. After a mixed solution of 200 kg of liquid paraffin and 20 kg of sorbitan isostearate as an emulsifier was added thereto, the mixture was stirred at room temperature to produce a water-in-oil emulsion.

Particles were formed by slowly adding each 200 kg of aqueous solutions containing 1% by weight, 2% by weight, 5% by weight, and 10% by weight of acetic acid thereto while being careful not to break the particles.

Aqueous solutions containing 1% by weight, 2% by weight, 5% by weight, 10% by weight, and 20% by weight of hydrochloric acid were each slowly added thereto to adjust the pH of the final solutions to 5 to 7 so that the particles were solidified hard. Only precipitated silica particles were taken, washed, dried, and sieved to prepare nano-free silica. As a result of photographing the prepared silica particles into a scanning electron micrograph, a nano-free state was shown.

**[Table 1]**

| | Example 1 | Example 2 |
|---|---|---|
| Average particle size (µm) | 7.2 | 8.5 |
| Oil absorption amount (ml/g) | 1.35 | 1.43 |
| Final solution pH | 6.5 | 6.1 |
| Friction coefficient (gf) | 142.3 | 131.2 |
| Avalanche Energy (mj/kg) | 14.5 | 13.8 |

As shown in Table 1, it was confirmed that the silica particles prepared according to the present invention were nano-free particles. In addition, it was confirmed that Example 2, in which the weak acid and the strong acid were inputted in a state that the concentrations of the weak acid and the strong acid were sequentially increased when inputting the weak acid and the strong acid, had more excellent physical properties than Example 1.

### [Industrial Applicability]

The technical problem to be achieved by the present invention is to solve the conventional problems, and an object of the present invention to provide nano-free silica spherical particles which can prevent nano harmfulness while preparing spherical silica particles for cosmetics with a diameter of 1 to 30 µm using water glass, and which can improve the feeling of use of cosmetics due to low friction coefficient and improved flowability of cosmetics, and a method for preparing the same.

## Claims

1. Silica particles, as spherical silica for cosmetics having a diameter of 1 to 30 µm obtained using water glass, **characterized in that** they are nano free as a result of measurement with a scanning electron microscope (SEM).

2. The silica particles of claim 1, wherein the silica particles have a friction coefficient (gf) of 160 gf or less, or an Avalanche Energy of 20 mj/kg or less.

3. A method for preparing silica particles, the method comprising:
a first step of preparing a mixed solution by adding an oil and an emulsifier to water glass or a mixture of water glass and water and stirring the mixture;
a second step of adding a weak acid or a weak acid aqueous solution to the mixed solution to perform reaction;
a third step of adding a strong acid aqueous solution to a reaction product of the second step and performing reaction; and
a fourth step of drying and pulverizing a resulting product of the third step.

4. The method of claim 3, wherein the oil is liquid paraffin, light oil, or kerosene.

5. The method of claim 3, wherein the emulsifier has a Hydrophilic Lipophilic Balance (HLB) of 4 to 6.

6. The method of claim 3, wherein the weak acid is one or more selected from the group consisting of acetic acid, carbonic acid, citric acid, and lactic acid.

7. The method of claim 3, wherein the addition of the weak acid aqueous solution is to sequentially input two or more aqueous solutions having different concentrations from a lower concentration one.

8. The method of claim 3, wherein the addition of the strong acid aqueous solution is to sequentially input two or more aqueous solutions having different concentrations from a lower concentration one.

9. Silica particles prepared by the method for preparing silica particles described in any one of claims 3 to 8.

10. Cosmetics containing the silica particles described in claim 1.
